(19) Europäisches Patentamt
European Patent Office
Office européen des brevets

(11) **EP 4 613 338 A1**

(12) **EUROPEAN PATENT APPLICATION**
published in accordance with Art. 153(4) EPC

(43) Date of publication:
**10.09.2025 Bulletin 2025/37**

(21) Application number: **23885676.9**

(22) Date of filing: **27.10.2023**

(51) International Patent Classification (IPC):
***A61Q 1/14*** (2006.01)   ***A61K 8/39*** (2006.01)

(52) Cooperative Patent Classification (CPC):
**A61K 8/39; A61Q 1/14**

(86) International application number:
**PCT/JP2023/038904**

(87) International publication number:
**WO 2024/095920 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.10.2022 JP 2022175111**

(71) Applicant: **Kao Corporation
Chuo-ku,
Tokyo 103-8210 (JP)**

(72) Inventors:
• **KAGAYA, Mariko**
  **Tokyo 131-8501 (JP)**
• **NAGASAKI, Yuko**
  **Tokyo 131-8501 (JP)**
• **UEYAMA, Chihiro**
  **Tokyo 131-8501 (JP)**
• **TAJIMA, Hitoshi**
  **Tokyo 131-8501 (JP)**
• **TAKEDA, Kosuke**
  **Wakayama-shi, Wakayama 640-8580 (JP)**

(74) Representative: **Hoffmann Eitle
Patent- und Rechtsanwälte PartmbB
Arabellastraße 30
81925 München (DE)**

(54) **METHOD FOR REMOVING OILY SOIL FROM SUBJECT SURFACE**

(57)    A method for removing oily stains from a subject surface, including bringing a composition containing the following components (A) and (B): (A) from 5 to 50 mass% of a nonionic surfactant having an HLB value of from 7 to 14 and/or a log P value of from 2.5 to 10.8 and (B) from 50 to 95 mass% of an oil phase having a viscosity of 15 mPa·s at 25°C, into contact with the oily stains on the subject surface, and performing rinsing with water or hot water without mixing together the composition and the oily stains on the subject surface.

EP 4 613 338 A1

**Description**

Field of the Invention

The present invention relates to a method for removing oily stains from a subject surface.

Background of the Invention

Cleaning is the removal of stains from a base (a surface of the skin, clothing or the like, or a hard surface) on which the stains are attached. For example, in cleaning of the body in a bathroom, laundering of clothing, and dishwashing, a cleansing agent is often used to perform cleaning, and finally washed off with water and removed. Here, oily stains (hereinafter, also referred to simply as stains) having low water solubility and generated in life, such as sebum and food oil, are removed using a composition mainly composed of a surfactant as a cleansing agent, and water. This involves a process in which a cleansing composition is applied, followed by, for example, rubbing of skin with a towel, rotation of clothing in a laundry machine, or scrub of dishes with sponge, which requires applying a physical force. That is, cleaning requires a physical force.

In more details, the process of cleaning comprises the three steps of (1) applying a cleansing agent to stains (bringing a cleansing agent into contact with stains), (2) applying a physical force to transfer stains attached on a base into the cleansing liquid, and (3) performing rinsing with water (including hot water; the same applies hereinafter) to remove a mixture of stains and the cleansing liquid from the base. Since mere contact of the cleansing agent with stains in step (1) does not detach the stains from the base, it is necessary to emulsify and disperse the stains in the cleansing liquid by applying a physical force in step (2). The step (2) is so-called a cleaning step, whose detergency is determined by how much stains transfer into the cleansing liquid.

This situation also applies to makeup removers of wash-off type. In makeup removers (cleansing agents), an oily agent is used as a main base agent because target makeup (stains) is an artificially made component having high hydrophobicity (extremely low water solubility), and thus cannot be removed with a composition comprising a surfactant and water in many cases. The removal with any of these agents unexceptionally requires a physical force in the cleaning step (2).

The application of a physical force in the cleaning step (2) of removing makeup matches the general expression "give a massage", "blended with makeup", "blended with makeup by circular movement", "blended with stains", "wash a face" or the like, and has become popular in the world as a generally well-known method.

For example, Non Patent Literature 1 is written on the assumption that removal of makeup involves blending. Non Patent Literature 2 describes blending as a proper method for removing makeup. From these, it can be seen that to remove the makeup, the need to blend makeup and a makeup remover after bringing the agents into contact with the makeup is widely known to the public.

That is, the method is widely recognized not only as a physicochemical need, but also as a practice of removing makeup in real life. In other words, there is a major assumption in the world that the action of blending is made in removal of makeup.

On the other hand, this method for removal has problems.

Actually, when a comparison is made between cases where a makeup remover is added dropwise to a model skin base coated with foundation and dried, and washed off with water after a physical force is applied by moving a finger in a circular pattern on the base and where nothing is done before the applied makeup remover is washed off with water, the visual evaluation of makeup removal completeness shows that with a common oil preparation, makeup is removed when the physical force is applied, but makeup is not removed at all when the physical force is not applied. This means that mere contact of the makeup remover and makeup does not transfer makeup as stains into the makeup remover.

Thus, it can be seen that with an existing makeup remover, makeup is removed only by applying a physical force.

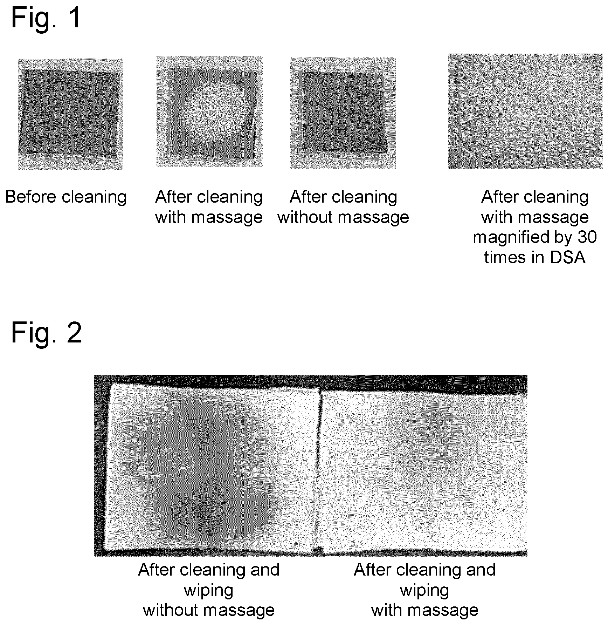

More specifically, 0.03 g of a long-lasting foundation (manufactured by Revlon Inc., ColorStay Makeup Foundation Mocha) is applied to a $10 \times 5$ cm$^2$ area of a model sheet having pore-like fine recesses (manufactured by Okamoto Kaseihin Co., Ltd., artificial leather, Laforet White), and dried for 1 hour or more. The foundation applied site is cut into small pieces of $2 \times 2.5$ cm$^2$, which are used as test pieces. A drop of a makeup remover (manufactured by Kao Corporation, Biore Perfect Oil) is applied onto the test piece with a dropper, and massaged by circularly moving a finger 10 times to the right and 10 times to the left under a load of 20 g (with massage) or left to stand (without massage). Thereafter, the makeup remover is washed off with tap water until there is no visual change, and drying is performed, followed by observation. Magnified pictures are taken by Digital Microscope VHX-5000 manufacture by KEYENCE CORPORATION. The results are shown in Fig. 1.

The results show that makeup in the portions of recesses is hardly removed even by applying a physical force. This may be because it is not possible to apply a physical force by direct contact of a finger with the inside of the recess, and as a result, makeup does not transfer into the makeup remover.

Next, an example is given in which an actual face is used. A foundation is applied to the entire face, and dried. Thereafter, the detergency is evaluated for cases where a makeup remover is taken in hand, spread over the face, thoroughly blended with makeup and washed off with water and where the makeup remover is taken in hand, spread over the face, and only washed off with water. Except for whether blending is performed or not, detailed instructions on how to apply, blend and rinse the makeup remover are not given. For the makeup removal completeness, the cleaning is followed by wiping the entire face with a sheet makeup remover, and visually evaluating the amount of the attached foundation.

More specifically, 0.26 g of a long-lasting foundation (manufactured by Revlon Inc., ColorStay Makeup Foundation Mocha) is applied to the entire face, and dried for 1 hour or more. Then 2.5 mL of the makeup remover (manufactured by Kao Corporation, Biore Perfect Oil) is taken in hand, and the makeup is removed by the specified method. After completion of the removal of makeup, water on the face is lightly wiped off, the entire face is wiped with a sheet makeup remover (manufactured by Kao Corporation, Biore Fukudake Cotton Uruoi Rich), and the sheet is observed. The results are shown in Fig. **2**.

In the case where the makeup remover is taken in hand, spread over the face, and only wiped off with water, a large amount of the foundation remains, and thus, the amount of the foundation attached to the sheet is large.

It can be seen that when an actual face is used, similarly to the model system, the physical force of blending makeup and the makeup remover is required, and makeup cannot be sufficiently removed when the makeup remover is only taken in hand and spread. In all of the step of taking the makeup remover and spreading the makeup remover, the step of blending the makeup remover and the step of rinsing the makeup remover with water, the face is touched by the hand to apply some physical force, but elimination of the blending step leads to a considerable decrease in detergency. Thus, it can be seen that the detergency significantly depends on the blending step.

Recently, due to the global warming and the mask-wearing life under the epidemic of infectious disease, long-lasting makeup which is unlikely to fall has come to be preferred. On the other hand, makeup removers have been faced with the growing desire not to rub the skin from the viewpoint of preventing damage to the skin. The action of removing makeup by users who do not want to rub the skin has made a transition to a short time and low friction. Under such circumstances, even with an oil-type makeup remover which is said to have high detergency, makeup may be left without being fully removed in the recess portions in facial features such as those around the eyes and the nose, or in the pores, which is a dissatisfactory point for users. On the other hand, for overcoming the failure to fully remove makeup, it is necessary that the face is massaged in every detail over time to blend the makeup remover and the makeup, which consumes time and labor, and causes friction against the skin, thus being a satisfactory point for users. That is, many people may be dissatisfied due to the dilemma between skin rubbing and makeup removal completeness, and the consumed time and labor for the makeup remover.

In such circumstances, studies have been made on a composition which is novel from the viewpoint of reducing the number of times of blending.

For example, Patent Literature 1 discloses an emulsified cleansing cosmetic which comprises liquid hydrocarbon oil, two specific nonionic surfactants, an acylated amino acid-type surfactant, and water, is rapidly blended with makeup, and has high cleansing power, and good stability over days.

Patent Literature 2 discloses a cleansing agent which comprises a paraffin mixture having a specific boiling point range, a specific nonionic surfactant, and a dihydric alcohol, is rapidly blended with makeup stains, and easily washed off, and gives a comfortable feeling after washing, an emollient feeing after washing, and a continued moist feeling after washing.

A composition which is novel from the viewpoint of reducing friction against the skin during massage has also been proposed.

For example, Patent Literature 3 discloses an oily cleansing agent with no feeling of incomplete removal of oil after water washing, comprising a specific nonionic surfactant and an oily agent. The oily cleansing agent has excellent usability such that it does not have a reduced viscosity, does not fall from the hand upon application, and is easily applied to an intended site. The oily cleansing agent has a good use impression such that it well spreads during cleansing, and can be felt moderately thick. The oily cleansing agent can be used in an environment where the hand and the face are wet, such as a washing stand or a bathroom. The oily cleansing agent has high detergency.

(Non Patent Literature 1) J. Soc. Comet. Chem. Japan. Vol. 25, No. 3 1991
(Non Patent Literature 2) VoCE, October 2022 issue, Kodansha Ltd.

(Patent Literature 1) JP-A-2017-100980
(Patent Literature 2) JP-A-2014-47141
(Patent Literature 3) JP-A-2006-22004

Summary of the Invention

The present invention relates to a method for removing oily stains from a subject surface, comprising bringing a composition comprising the following components (A) and (B):

(A) a nonionic surfactant having an HLB value of from 7 to 14 and/or a log P value of from 2.5 to 10.8, and
(B) an oil phase having a viscosity of 15 mPa·s at 25°C

into contact with the oily stains on the subject surface, and performing rinsing with water or hot water without mixing together the composition and the oily stains on the subject surface.

Brief Description of Drawings

Fig. 1 illustrates detergency when a makeup remover is added dropwise to a model skin base coated with a foundation and dried.
Fig. 2 illustrates a difference in detergency depending on whether blending is performed or not when a foundation is applied to the entire face and dried, followed by application of a makeup remover.

Detailed Description of the Invention

For the cleansing cosmetics disclosed in Patent Literature 1 and 2, makeup is rapidly blended, and even by light massage, the agents and the makeup are mixed to remove the makeup, but the requirement of so-called a blending step is assumed, it is not possible to remove makeup in pores which cannot be accessed by the hand or in portions which the user has failed to massage, and thus, dissatisfactory points for users are not overcome.

The cleansing agent disclosed in Patent Literature 3 can be felt thick to reduce irritation to the skin and discomfort during use. On the other hand, it is assumed that removal is performed only by giving a massage, time and labor are consumed, friction against the skin does not disappear, and it is also not possible to remove makeup in portions which the user has failed to massage.

Accordingly, a cleaning method capable of thoroughly removing makeup without applying the physical force of blending makeup and a makeup remover in removal of makeup on the skin is desired. Not only makeup removers but also other cleansing agents are faced with the desire to thoroughly remove stains without applying a physical force.

The present inventors found that by bringing a composition comprising a specific nonionic surfactant and an oil phase having a low viscosity into contact with oily stains such as a cosmetic, the stains can be thoroughly removed without mixing the composition and the stains.

According to the present invention, even hardly removable oily stains such as a foundation can be thoroughly removed without applying an unnecessary physical force.

The component (A) for use in the present invention is a nonionic surfactant having an HLB value of from 7 to 14 and/or a log P value of from 2.5 to 10.8.

Here, the HLB value is an index of hydrophile lipophile balance, and is herein determined by the following Griffin's equation.

$$\text{HLB} = 20 \times \frac{\text{molecular weight of hydrophilic moiety}}{\text{molecular weight of surfactant}}$$

The log P value is a logarithmic value of a 1-octanol/water partition coefficient, and means, when a compound is dissolved as a solute in two-liquid phase solvent system of 1-octanol and water, a ratio of the equilibrium concentrations of the solute in the respective solvents at partition equilibrium. The log P value is commonly expressed in the form of the logarithm log P where the base is 10.

The log P value is calculated by, for example, equation (1) using a chemical potential μ* determined on the basis of the COSMO-RS theory presented by Klamt et al. (Journal of Computer-Aided Molecular Design, 15, 355-365, 2001) as a predicted value with BIOVIA COSMO therm (Dassault Systemes).

$$\log P = \frac{0.4343}{RT}\left[\mu^{*X}_O - \mu^{*X}_W + RT\ln\frac{v_O}{v_W}\right] \quad (1)$$

Here, v is a molar volume of a solvent, R is the gas constant, and T is an absolute temperature. The subscript symbols X, O and W represent a solute, 1-octanol and water, respectively. The surface charge distributions of the molecules of the solute and the solvent, respectively, which are required to obtain $\mu^*$, are obtained by DFT computation (BP/def-TZVP) with BIOVIA TURBOMOLE (Dassault Systemes).

Such a nonionic surfactant has moderate solubility in water and oil, is not excessively dissolved in rinse water and the oily agent during rinsing with water, and acts on the skin (subject surface) to draw water between stains and the skin, so that the stains can be separated from the skin and dispersed in the water. This may be because surface modification which is hydrophilization of a solid surface as a subject is performed to improve the water wettability of the subject surface.

In this respect, the HLB value is preferably from 7 **to 12.9.** The log P value is preferably from 2.5 to 8.5, and more preferably from 4 to 8.

An HLB value of less than 7 leads to excessively high solubility of the surfactant in oil, and an HLB value of more than 14 leads to excessively high solubility in water.

A log P value of less than 2.5 leads to an excessively high partition ratio of the surfactant in oil, and a log P value of more than 10.8 leads to an excessively high partition ratio in water.

The nonionic surfactants as the component (A) can be used in combination of two or more thereof, but a nonionic surfactant having an HLB value of less than 7 and a nonionic surfactant having an HLB value of more than 14, which are combined such that a value obtained by taking the arithmetic average of the HLB values of the respective nonionic surfactants based on their mass ratio is from 7 to 14, cannot be used.

The nonionic surfactant as the component (A) is not limited as long as it is used for common cleansing compositions, and examples thereof include polyoxyethylene alkyl fatty acid esters, polyoxyethylene alkyl ethers, polyoxyethylene sorbitan fatty acid esters, polyoxyethylene sorbitol fatty acid esters, polyoxyethylene glycerin fatty acid esters, polyglycerin fatty acid esters, sorbitan fatty acid esters, alkylglucosides, and alkyl glyceryl ethers.

Of these, polyoxyethylene alkyl ethers, polyoxyethylene sorbitan fatty acid esters, polyoxyethylene sorbitol fatty acid esters, polyoxyethylene fatty acid esters, polyoxyethylene glycerin fatty acid esters, and polyglycerin fatty acid esters are preferable from the view point of surface modification.

The polyoxyethylene alkyl ethers preferably have an alkyl group having 8 to 22 carbon atoms, and 2 to 20 moles of an oxyethylene group added, more preferably have an alkyl group having 12 to 18 carbon atoms, and 3 to 12 moles of an oxyethylene group added, and preferably have an alkyl group selected from the group consisting of a linear alkyl group having 12 to 16 carbon atoms, an unsaturated alkyl group having 18 carbon atoms, and a branched alkyl group having 12 to 18 carbon atoms.

The polyoxyethylene sorbitan fatty acid esters or the polyoxyethylene sorbitol fatty acid esters preferably have an alkyl group having 8 to 22 carbon atoms, and 6 to 60 moles of an oxyethylene group added, more preferably have 3 unsaturated chain alkyl groups, and 20 to 40 moles of an oxyethylene group added, or 4 unsaturated chain alkyl groups, and 35 to 40 moles of an oxyethylene group added, or 3 to 4 saturated branched chain alkyl groups, and 20 to 40 moles of an oxyethylene group added, and further more preferably have an alkyl group having 18 carbon atoms.

The polyoxyethylene fatty acid esters preferably have 1 to 2 alkyl groups having 8 to 22 carbon atoms, and 2 to 35 moles of an oxyethylene group added, more preferably have 1 to 2 alkyl groups having 12 to 18 carbon atoms, and 3 to 12 moles of an oxyethylene group added, and further more preferably have one alkyl group having 14 to 18 carbon atoms, and 3 to 5 or 7 to 9 moles of an oxyethylene group added, or one alkyl group having 12 to 16 carbon atoms, and 10 to 12 moles of an oxyethylene group added, or 2 alkyl groups having 12 to 18 carbon atoms, and 3 to 12 moles of an oxyethylene group added, and even more preferably have 2 alkyl groups having 12 to 18 carbon atoms, and 5 to 12 moles of an oxyethylene group added. The polyoxyethylene fatty acid esters preferably have a linear alkyl group having 12 carbon atoms, or one or more branched alkyl groups having 18 carbon atoms.

The polyoxyethylene glycerin fatty acid esters preferably have an alkyl group having 8 to 22 carbon atoms, and 1 to 50 moles of an oxyethylene group added, more preferably have 1 to 3 alkyl groups having 12 to 18 carbon atoms, and 6 to 30 moles of an oxyethylene group added, and further more preferably have one alkyl group having 12 to 18 carbon atoms, and 6 or 10 to 30 moles of an oxyethylene group added, or 2 or 3 alkyl groups having 12 to 18 carbon atoms, and 6 to 30 moles of an oxyethylene group added. The polyoxyethylene glycerin fatty acid esters preferably have a branched alkyl group having 18 carbon atoms.

The polyglycerin fatty acid esters preferably have an alkyl group having 8 to 22 carbon atoms, and polyglycerin having an average polymerization degree of from 2 to 80, and more preferably are polyglycerin fatty acid esters which have an alkyl group having 10 to 14 carbon atoms and in which the ratio of the polymerization degree to the number of fatty acids bonded (polymerization degree of polyglycerin / number of fatty acids bonded) is from 2 to 5.

The components (A) can be used alone, or in combination of two or more thereof, and the content thereof is preferably from 5 to 50 mass%, more preferably from 9 to 40 mass%, and further more preferably from 10 to 30 mass%, in the composition, from the viewpoint of obtaining good makeup dispersing properties and detergency.

The component (B) is an oil phase having a viscosity of 15 mPa·s or less at 25°C.

The component (B) has a function of rapidly dissolving an oil-soluble polymer which forms a hard film on the surface of a foundation coated film, entering the coated film, and softening the coated film itself to impart flowability, and a role of delivering the component (A) to the surface of the skin (base).

The oily agent forming the oil phase as the component (B) is not limited as long as it is liquid oil which is commonly used for cosmetics, and examples thereof include hydrocarbon oil such as isododecane, isohexadecane, light liquid isoparaffin, liquid isoparaffin and liquid paraffin; ether oil such as dicapryl ether and alkyl-1,3-dimethylbutyl ether; monoester oil such as cetyl 2-ethylhexanoate, cetyl octanoate, isononyl isononanoate, isotridecyl isononanoate, hexyl laurate, isopropyl myristate, octyldodecyl myristate, myristyl myristate, 2-hexyldecyl myristate, isopropyl palmitate and octyl palmitate; diester oil such as propylene glycol dicaprate and neopentyl glycol dicaprate; triester oil such as tri(capryl/capric acid) glycerin, glycerin trioleate and glycerin tri-2-ethylhexanoate; ether oil such as dicapryl ether, cetyl-1,3-dimethyl butyl ether; and plant oil such as oil of olive and jojoba oil.

For the component (B), oily agents may be used alone, or in combination of two or more thereof. A viscosity of two or more oily agents used in combination is obtained by measuring the viscosity of a mixture of the oily agents.

The viscosity is measured at 60 rpm (revolutions/min) for 60 seconds with spindle No. 1 or at 30 rpm (revolutions/min) for 60 seconds with spindle No. 1 using, for example, a Brookfield viscometer (Vismetron Viscometer: Model VS-A1 (manufactured by Shibaura System Co., Ltd.)).

The content of the component (B) is preferably from 50 to 95 mass%, and more preferably from 70 to 90 mass%, in the composition, from the viewpoint of obtaining good detergency and dispersing properties.

The composition for use in the present invention may comprise, in addition to the above-described components, components used for common cleansing compositions, for example, a surfactant other than the component (A), a polymer compound, powder, water, a moisturizer, a bactericide, an alcohol, an anti-inflammatory agent, an antiseptic agent, a chelating agent, a salt, a pearling agent, a perfume, a chilling agent, a pigment, an ultraviolet absorber, an antioxidant, a plant extract and the like.

The composition for use in the present invention can be produced by mixing and stirring the components using a common method, and can be used as a cleansing composition. The composition of the present invention can be preferably used for a cleansing agent to remove oily stains from a solid surface (a surface of the human skin, clothing or the like, or a hard surface), and is preferably used for cleaning the skin, and particularly preferably used as, for example, a face wash, a makeup remover, or a whole-body wash for removing oily stains such as sunscreens.

In the present invention, the above-described composition is brought into contact with a cosmetic such as makeup, whereby the cosmetic can be removed without mixing together the composition and the cosmetic.

The composition can be brought into contact with the cosmetic by, for example, filling a container with the composition, which is dropped onto the cosmetic; taking an appropriate amount of the composition in hand, followed by placement on the cosmetic; or filling a mist dispenser or an aerosol container with the composition, which is sprayed to the cosmetic.

After being brought into contact with the cosmetic, the composition is left to stand if necessary, and then washed (rinsed) off with water or hot water in the shower or the like, whereby the cosmetic can be removed. The mixed composition and cosmetic are transferred into water under running water or the like, and thus removed from the skin. The temperature of the water or hot water is, for example, from 5 to 45°C. The time of standing after the composition is brought into contact with the cosmetic may be arbitrary, for example, from 10 seconds to 20 minutes, preferably from 20 seconds to 3 minutes.

Conventionally, the action mechanism of common oil-type makeup removers for hardly removable long-lasting makeup among target makeups (stains) comprises a process in which a tough surface comprising an oil-soluble polymer (trimethylsiloxysilicate or the like) forming a long-lasting makeup coated film is dissolved by the composition under the physical force of, for example, finger blending to loosen the makeup, and a process in which mixing with water by hand during rinsing makes the surfactant emulsify the oily agent in which the makeup is dispersed, thereby separating the makeup from the skin. That is, involvement of a physical force with a human hand is required for dissolving and dispersing the makeup coated film.

In the present invention, mere contact with makeup leads to spontaneous infiltration into the coated film in the absence of a physical force, and exposure to a water flow, which is not accompanied by rubbing, allows the makeup to be dispersed from pore-like small recesses, and washed off.

Accordingly, with the conventional oil-type makeup remover, application of the composition to a cosmetic such as makeup is required to be followed by applying the physical force of, for example, giving a massage or blending with the cosmetic, and thus mixing the composition and the cosmetic to transfer the cosmetic into the composition, whereas in the present invention, the cosmetic can be removed without mixing together the composition and the cosmetic.

As described above, the conventional cleaning process involves the three steps of (1) applying a cleansing agent to stains (bringing a cleansing agent into contact with stains), (2) applying a physical force to transfer stains attached on a base into the cleaning liquid, and (3) performing rinsing with water to remove a mixture of stains and the cleansing liquid from the base.

In the present invention, by only the steps (1) and (3), makeup can be dispersed even from pore-like small recesses, and thoroughly removed. This is the same not only for cosmetics such as makeup but also for other oily stains, and particularly significant for long-lasting foundations, for example.

For the embodiments described above, the present invention further discloses the following methods and compositions.

<1> A method for removing oily stains from a subject surface, comprising bringing a composition comprising the following components (A) and (B):

(A) a nonionic surfactant having an HLB value of from 7 to 14 and/or a log P value of from 2.5 to 10.8, and

(B) an oil phase having a viscosity of 15 mPa·s at 25°C

into contact with the oily stains on the subject surface, and performing rinsing with water or hot water without mixing together the composition and the oily stains on the subject surface.

<2> The method according to <1>, wherein in the composition, a content of the component (A) is from 5 to 50 mass%, and a content of the component (B) is from 50 to 95 mass%.

<3> The method according to <1> or <2>, wherein in the composition, the content of the component (A) is preferably from 9 to 40 mass%, and more preferably from 10 to 30 mass%.

<4> The method according to any one of <1> to <3>, wherein the HLB value of the component (A) is preferably from 7 to 12.9.

<5> The method according to any one of <1> to <4>, wherein the log P value of the component (A) is preferably from 2.5 to 8.5, and more preferably from 4 to 8.

<6> The method according to any one of <1> to <5>, wherein the component (A) comprises one or more selected from the group consisting of a polyoxyethylene alkyl ether, a polyoxyethylene sorbitan fatty acid ester or a polyoxyethylene sorbitol fatty acid ester, a polyoxyethylene fatty acid ester, a polyoxyethylene glycerin fatty acid ester and a polyglycerin fatty acid ester.

<7> The method according to any one of <1> to <6>, the component (A) comprising a polyoxyethylene alkyl ether, wherein the polyoxyethylene alkyl ether preferably has an alkyl group having 8 to 22 carbon atoms, and 2 to 20 moles of an oxyethylene group added, and more preferably has an alkyl group having 12 to 18 carbon atoms, and 3 to 12 moles of an oxyethylene group added.

<8> The method according to any one of <1> to <7>, wherein the component (A) comprises a polyoxyethylene alkyl ether having an alkyl group selected from the group consisting of a linear alkyl group having 12 to 16 carbon atoms, an unsaturated alkyl group having 18 carbon atoms, and a branched alkyl group having 12 to 18 carbon atoms.

<9> The method according to any one of <1> to <8>, the component (A) comprising a polyoxyethylene sorbitan fatty acid ester or a polyoxyethylene sorbitol fatty acid ester, wherein the polyoxyethylene sorbitan fatty acid ester or the polyoxyethylene sorbitol fatty acid ester preferably has an alkyl group having 8 to 22 carbon atoms, and 6 to 60 moles of an oxyethylene group added, and more preferably has 3 unsaturated chain alkyl groups, and 20 to 40 moles of an oxyethylene group added, or 4 unsaturated chain alkyl groups, and 35 to 40 moles of an oxyethylene group added, or 3 or 4 saturated branched chain alkyl groups, and 20 to 40 moles of an oxyethylene group added.

<10> The method according to any one of <1> to <9>, the component (A) comprises a polyoxyethylene fatty acid ester, wherein the polyoxyethylene fatty acid ester preferably has 1 or 2 alkyl groups having 8 to 22 carbon atoms, and 2 to 35 moles of an oxyethylene group added, more preferably has 1 or 2 alkyl groups having 12 to 18 carbon atoms, and 3 to 12 moles of an oxyethylene group added, and further more preferably has one alkyl group having 14 to 18 carbon atoms, and 3 to 5 or 7 to 9 moles of an oxyethylene group added, or one alkyl group having 12 to 16 carbon atoms, and 10 to 12 moles of an oxyethylene group added, or 2 alkyl groups having 12 to 18 carbon atoms, and 3 to 12, preferably 5 to 12 moles of an oxyethylene group added.

<11> The method according to any one of <1> to <10>, wherein the component (A) comprises a polyoxyethylene fatty acid ester having one or more linear alkyl groups having 12 carbon atoms, or branched alkyl groups having 18 carbon atoms.

<12> The method according to any one of <1> to <11>, the component (A) comprises a polyoxyethylene glycerin fatty acid ester, wherein the polyoxyethylene glycerin fatty acid ester preferably has an alkyl group having 8 to 22 carbon atoms, and 1 to 50 moles of an oxyethylene group added, more preferably has 1 to 3 alkyl groups having 12 to 18 carbon atoms, and 6 to 30 moles of an oxyethylene group added, and further more preferably has one alkyl group having 12 to 18 carbon atoms, and 6 or 10 to 30 moles of an oxyethylene group added, or 2 or 3 alkyl groups having 12 to 18 carbon atoms, and 6 to 30 moles of an oxyethylene group added.

<13> The method according to any one of <1> to <12>, wherein the component (A) comprises a polyoxyethylene glycerin fatty acid ester having a branched alkyl group having 18 carbon atoms.

<14> The method according to any one of <1> to <13>, the component (A) comprises a polyglycerin fatty acid ester, wherein the polyglycerin fatty acid ester preferably has an alkyl group having 8 to 22 carbon atoms, and polyglycerin having an average polymerization degree of from 2 to 80, and more preferably is a polyglycerin fatty acid ester which has an alkyl group having 10 to 14 carbon atoms and in which a ratio of the polymerization degree to the number of fatty acids bonded (polymerization degree of polyglycerin / number of fatty acids bonded) is from 2 to 5.

<15> The method according to any one of <1> to <14>, wherein in the composition, the content of the component (B) is preferably from 70 to 90 mass%.

<16> The method according to any one of <1> to <15>, wherein the oily stains comprise a cosmetic.

<17> The method according to <16>, wherein the cosmetic comprises a foundation.

<18> The method according to any one of <1> to <17>, wherein the subject surface is hydrophobic.

<19> The method according to any one of <1> to <18>, wherein the subject surface is human skin.

<20> The method according to any one of <1> to <19>, wherein the composition is filled in a container equipped with a mist dispenser or an aerosol container, for use in spraying on the skin.

<21> The method according to any one of <1> to <20>, wherein the subject surface is rinsed with water or hot water after a lapse of an arbitrary time of from 10 seconds to 20 minutes, preferably from 20 seconds to 3 minutes, after the composition is brought into contact with the oily stains on the subject surface.

<22> The method according to any one of <1> to <21>, wherein the component (A) in the composition brought into contact with the oily stains on the subject surface infiltrates into the oily stains with the component (B), and makes the subject surface hydrophilic, and the subject surface is then rinsed with water or hot water to remove the oily stains from the subject surface.

<23> A composition comprising the following components (A) and (B):

(A) from 5 to 50 mass% of a nonionic surfactant having an HLB value of from 7 to 14 and/or a log P value of from 2.5 to 10.8, and
(B) from 50 to 95 mass% of an oil phase having a viscosity of 15 mPa·s at 25°C,

wherein the component (A) infiltrates into target oily stains on a solid surface with the component (B), and makes the subject surface hydrophilic.

<24> A composition comprising the following components (A) and (B):

(A) from 5 to 50 mass% of a nonionic surfactant having an HLB value of from 7 to 14 and/or a log P value of from 2.5 to 10.8, and
(B) from 50 to 95 mass% of an oil phase having a viscosity of 15 mPa·s at 25°C,

wherein the component (A) infiltrates into target oily stains on a solid surface with the component (B), and modifies the subject surface to improve water wettability of the subject surface.

<25> A composition comprising the following components (A) and (B):

(A) from 5 to 50 mass% of a nonionic surfactant having an HLB value of from 7 to 14 and/or a log P value of from 2.5 to 10.8, and
(B) from 50 to 95 mass% of an oil phase having a viscosity of 15 mPa·s at 25°C,

wherein the component (A) infiltrates into target oily stains on a solid surface with the component (B), and modifies the subject surface to draw water between the subject surface and the oily stains.

<26> The composition according to any one of <23> to <25>, wherein in the composition, a content of the component (A) is preferably from 9 to 40 mass%, and more preferably from 10 to 30 mass%.

<27> The composition according to any one of <23> to <26>, wherein the HLB value of the component (A) is preferably from 7 to 12.9.

<28> The composition according to any one of <23> to <27>, wherein the log P value of the component (A) is preferably from 2.5 to 8.5, and more preferably from 4 to 8.

<29> The composition according to any one of <23> to <28>, wherein in the composition, a content of the component (B) is preferably from 70 to 90 mass%.

<30> The composition according to any one of <23> to <29>, wherein the oily stains comprise a cosmetic.

<31> The composition according to <30>, wherein the cosmetic comprises a foundation.

<32> The composition according to any one of <23> to <31>, wherein the subject surface is hydrophobic.

<33> The composition according to any one of <23> to <32>, wherein the subject surface is human skin.

<34> The composition according to any one of <23> to <33>, wherein the composition is filled in a container with a mist dispenser or an aerosol container, for use in spraying on the skin.

Examples

Examples 1 to 47 and Comparative Examples 1 to 8

Cleansing compositions whose formulations are shown in Tables 1 to 7 were produced, and their performance to remove a cosmetic (foundation) was evaluated. The results are also shown in Tables 1 to 7.

(Production method)

The components were weighed, and mixed and stirred while being heated if necessary, thereby producing the cleansing composition.

(Evaluation method)

Performance to remove cosmetic:

A long-lasting foundation (manufactured by Revlon Inc., ColorStay Makeup Foundation Mocha) was applied in an amount of 0.03 g to a $10 \times 5$ cm$^2$ area of a model sheet having pore-like fine recesses (manufactured by Okamoto Kaseihin Co., Ltd., artificial leather, Laforet White), and dried for 1 hour or more. The foundation applied site was cut into small pieces of $2 \times 2.5$ cm$^2$, which were used as test pieces. A drop of a makeup remover was applied onto the test piece with a dropper, and left to stand for 1 minute. Thereafter, the makeup remover was washed off with tap water until no visual change was observed, followed by drying. The foundation removal completeness was visually evaluated by the following criteria.

A; The foundation coated film is fully removed.
B; The foundation coated film is almost removed.
C; The foundation coated film is apparently removed.
D; The foundation coated film is slightly removed, and mostly remains.
E; The foundation remains unchanged.

[Table 1]

| | Name of component | Name of raw material | Name of Manufacturer | HLB | logP | Example | | | | | | |
| --- | --- | --- | --- | --- | --- | --- | --- | --- | --- | --- | --- | --- |
| | | | | | | 1 | 2 | 3 | 4 | 5 | 6 | 7 |
| (A) | Laureth-3 | EMULGEN 103 | Kao Corporation | 8.1 | 5.6 | 20 | | | | | | |
| | Ceteareth-3 | EMALEX 103 | Nihon Emulsion Co., Ltd. | 7.1 | 7.7 | | 20 | | | | | |
| | Oleth-5 | EMULGEN 404 | Kao Corporation | 8.8 | 7.5 | | | 20 | | | | |
| | Oleth-6 | EMULGEN 408 | Kao Corporation | 10 | 7.4 | | | | 20 | | | |
| | (C11-15) Pareth-5 | EMULGEN 705 | Kao Corporation | 10.5 | 5.4 | | | | | 20 | | |
| | Laureth-6 | EMULGEN 106 | Kao Corporation | 11.7 | 5.4 | | | | | | 20 | |
| | Oleth-9 | EMULGEN 409PV | Kao Corporation | 12 | 7.3 | | | | | | | 20 |

(continued)

| | | Name of component | Name of raw material | Name of Manufacturer | HLB | logP | Example | | | | | | |
|---|---|---|---|---|---|---|---|---|---|---|---|---|---|
| | | | | | | | 1 | 2 | 3 | 4 | 5 | 6 | 7 |
| (B) | | Isododecane (viscosity; 3mPa·s) | MARUKASOL R | Maruzen Petrochemical Co., Ltd. | | | | 80 | 80 | 80 | 80 | 80 | 80 |
| | | Isopropyl myristate (viscosity; 6.6mPa·s) | EXCEPARL IPM | Kao Corporation | | | 80 | | | | | | |
| Performance to remove cosmetic | | | | | | | A | B | B | B | A | A | B |

[Table 2]

| | | Name of component | Name of raw material | Name of Manufacturer | HLB | logP | Example | | | | | | |
|---|---|---|---|---|---|---|---|---|---|---|---|---|---|
| | | | | | | | 8 | 9 | 10 | 11 | 12 | 13 | 14 |
| (A) | | (C11-15) Pareth7 | EMULGEN 707 | Kao Corporation | 12.1 | 5.2 | 20 | | | | | | |
| | | Ceteareth 7 | EMULGEN 210P | Kao Corporation | 12.9 | 6.6 | | 20 | | | | | |
| | | (C11-15) Pareth9 | EMULGEN 709 | Kao Corporation | 13.3 | 4.9 | | | 20 | | | | |
| | | Laureth-9 | EMULGEN 109P | Kao Corporation | 13.6 | 4.4 | | | | 20 | | | |
| | | PEG-3 isostearate | EMALEX PEIS-3 | Nihon Emulsion Co., Ltd. | 7 | 7.4 | | | | | 20 | | |
| | | PEG-8 dilaurate | EMALEX 400di-L | Nihon Emulsion Co., Ltd. | 8 | 10.7 | | | | | | 20 | |
| | | PEG-8 isostearate | EMALEX PEIS-8 | Nihon Emulsion Co., Ltd. | 10 | 7.0 | | | | | | | 20 |
| (B) | | Isododecane (viscosity; 3mPa·s) | MARUKASOL R | Maruzen Petrochemical Co., Ltd. | | | 80 | 80 | | | 80 | | 80 |
| | | Isopropyl myristate (viscosity; 6.6mPa·s) | EXCEPARL IPM | Kao Corporation | | | | | 80 | | | | |
| | | Dicaprylyl ether (viscosity; 5.4mPa·s) | CETIOL OE-DEO | BASF JAPAN LTD. | | | | | | 80 | | 80 | |
| Performance to remove cosmetic | | | | | | | B | B | C | C | B | \| B | B |

[Table 3]

| | Name of component | Name of raw material | Name of Manufacturer | HLB | logP | Example | | | | | | |
|---|---|---|---|---|---|---|---|---|---|---|---|---|
| | | | | | | 15 | 16 | 17 | 18 | 19 | 20 | 21 |
| (A) | PEG-12 laurate | EMANON 1112 | Kao Corporation | 13.7 | 3.9 | 20 | | | | | | |
| | PEG-12 Dilaurate | EMALEX 600di-L | Nihon Emulsion Co., Ltdn | 10 | 10.0 | | 20 | | | | | |
| | PEG-6 glyceryl isostearate | EMALEX GWIS-106 | Nihon Emulsion Co., Ltd. | 8 | 6.6 | | | 20 | | | | |
| | PEG-8 glyceryl isostearate | EMALEX GWIS-108 | Nihon Emulsion Co., Ltd. | 10 | 6.6 | | | | 20 | | | |
| | PEG-10 glyceryl isostearate | EMALEX GWIS-110 | Nihon Emulsion Co., Ltd. | 10 | 6.5 | | | | | 20 | | |
| | Coconut oil fatty acid PEG-7 glyceryl | LEVENOL C-301B | Kao Corporation | 13 | 5.0 | | | | | | 20 | |
| | Polyglyceryl-2 laurate | SUNSOFT Q-12D-C | Taiyo Kagaku Co., Ltd. | 8.5 | 5.0 | | | | | | | 20 |
| (B) | Isododecane (viscosity; 3mPa·s) | MARUKASOL R | Maruzen Petrochemical Co., Ltd. | | | 40 | | 80 | | 80 | | |
| | Isopropyl myristate (viscosity; 6.6mPa·s) | EXCEPARL IPM | Kao Corporation | | | 40 | | | | | 80 | |
| | Dicaprylyl ether (viscosity; 5.4mPa·s) | CETIOL OE-DEO | BASF JAPAN LTD. | | | | 80 | | 80 | | | 80 |
| | Performance to remove cosmetic | | | | | B | C | B | A | B | C | A |

[Table 4]

| | Name of component | Name of raw material | Name of Manufacturer | HLB | logP | Example | | | Comparative Example | | |
|---|---|---|---|---|---|---|---|---|---|---|---|
| | | | | | | 22 | 23 | 24 | 1 | 2 | 3 |
| (A) | Polyglyceryl-2 caprate | SUNSOFT Q-10D-C | Taiyo Kagaku Co., Ltd. | 9.5 | 4.0 | 20 | | | | | |
| | Eglyqlyceryl-10 trilaurate | SUNSOFT Q-123Y-C | Taivo Kagaku Co., Ltd. | 10.4 | 6.0 | | 20 | | | | |
| | Polyglyceryl-6 dicaprate | SUNSOFT Q-102H-C | Taiyo Kagaku Co., Ltd. | 10.2 | 2.6 | | | 20 | | | |

(continued)

| | Name of component | Name of raw material | Name of Manufacturer | HLB | logP | Example | | | Comparative Example | | |
|---|---|---|---|---|---|---|---|---|---|---|---|
| | | | | | | 22 | 23 | 24 | 1 | 2 | 3 |
| Other than (A) | PEG-6 caprylic/capric glycerides PEG-6 | TEGOSOFT GMC 6 MB | Evonik Operations GmbH | 15 | 2.4 | | | | 20 | | |
| | Polysorbate 20 | RHEODOL TW-L120 | Kao Corporation | 16.7 | 2.1 | | | | | 20 | |
| | Sorbitan sesquioleate | RHEODOL AO-15V | Kao Corporation | 3.7 | 16.2 | | | | | | 20 |
| (B) | Isododecane (viscosity; 3mPa·s) | MARUKASOL R | Maruzen Petrochemical Co., Ltd. | | | | 80 | | 80 | 80 | 80 |
| | Isopropyl myristate (viscosity; 6.6mPa·s) | EXCEPARL IPM | Kao Corporation | | | 80 | | | | | |
| | Dicaprylyl ether (viscosity; 5.4mPa·s) | CETIOL OE-DEO | BASF JAPAN LTD. | | | | | 80 | | | |
| Performance to remove cosmetic | | | | | | A | A | C | E | E | E |

[Table 5]

| | Name of component | Name of raw material | Name of Manufacturer | Comparative Example 4 | Example | | | | | | | | | | | Comparative Example 5 |
|---|---|---|---|---|---|---|---|---|---|---|---|---|---|---|---|---|
| | | | | | 25 | 26 | 27 | 28 | 29 | 30 | 31 | 32 | 33 | 34 | 35 | |
| (A) | Laureth-3 (HLB; 8.1, logP; 5.6) | EMULGEN 103 | Kao Corporation | 3 | 5 | 8 | 9 | 10 | 12 | 15 | 20 | 25 | 30 | 40 | 50 | 60 |
| (B) | Isododecane (viscosity; 3mPa·s) | MARUKASOL R | Maruzen Petrochemical Co., Ltd. | 97 | 95 | 92 | 91 | 90 | 88 | 85 | 80 | 75 | 70 | 60 | 50 | 40 |
| | Performance to remove cosmetic | | | E | C | C | B | B | A | A | A | A | B | B | C | D |

[Table 6]

| | Name of component | Name of raw material | Name of Manufacturer | Viscosity of oily agent (mPa·s) | Example | | | | | | | | | Comparative Example | |
|---|---|---|---|---|---|---|---|---|---|---|---|---|---|---|---|
| | | | | | 36 | 37 | 38 | 39 | 40 | 41 | 42 | 43 | 44 | 6 | 7 |
| (A) | Laureth-3 (HLB;8.1, logP;5.6) | EMULGEN 103 | Kao Corporation | - | 20 | 20 | 20 | 20 | 20 | 20 | 20 | 20 | 20 | 20 | 20 |
| (B) | Isododecane | MARUKASOL R | Maruzen Petrochemical Co., Ltd. | 3 | 80 | | | | | | | | | | |
| | Hydrogenated polyi-sobutene | PARLEAM 4 | NOF CORPORA-TION | 5.3 | | 80 | | | | | | | | | |
| | Hydrogenated polyi-sobutene | PARLEAM EX | NOF CORPORA-TION | 14 | | | 80 | | | | | | | | |
| | Isopropyl myristate | EXCEPARL IPM | Kao Corporation | 6.6 | | | | 80 | | | | | | | |
| | Isopropyl palmitate | EXCEPARL IPP | Kao Corporation | 10 | | | | | 80 | | | | | | |
| | Isononyl isononanoate | SARACOS 99 | The Nisshin OilliO Group, Ltd. | 7 | | | | | | 80 | | | | | |
| | Cetyl 2-ethylhexanoate | EXCEPARL HO | Kao Corporation | 14 | | | | | | | 80 | | | | |
| | Dicaprylyl ether | CETIOL OE-DEO | BASF JAPAN LTD. | 5.4 | | | | | | | | 80 | | | |
| | Cetyl-1,3-dimethyl butyl ether | ASE-166K | Kao Corporation | 8.2 | | | | | | | | | 80 | | |
| | Liquid paraffin | HICALL K-350 | KANEDA Co., Ltd. | 135 | | | | | | | | | | 80 | |
| | Tri(caprylic/capric) glyceryl | COCONAD MT | Kao Corporation | 23 | | | | | | | | | | | 80 |
| | Performance to remove cosmetic | | | | A | A | A | A | B | B | A | A | A | E | E |

**EP 4 613 338 A1**

[Table 7]

| | | Name of component | Name of raw material | Name of Manufacturer | Viscosity of oily agent (mPa·s) | Example | | | Comparative Example |
|---|---|---|---|---|---|---|---|---|---|
| | | | | | | 45 | 46 | 47 | 8 |
| (A) | | Laureth-3 (HLB; 8.1, logP; 5.6) | EMULGEN 103 | Kao Corporation | - | 20 | 20 | 20 | 20 |
| (B) | | Isododecane | MARUKASOL R | Maruzen Petrochemical Co., Ltd. | 3 | 60 | 40 | 30 | 25 |
| | | Liquid paraffin | HICALL K-350 | KANEDA Co., Ltd. | 135 | 20 | 40 | 50 | 55 |
| | | Viscosity of oil phase (mPa·s) | | | | 4.6 | 7.6 | 13.1 | 18.1 |
| | | Performance to remove cosmetic | | | | A | A | B | D |

## Claims

1. A method for removing oily stains from a subject surface, comprising bringing a composition comprising the following components (A) and (B):

   (A) a nonionic surfactant having an HLB value of from 7 to 14 and/or a log P value of from 2.5 to 10.8, and
   (B) an oil phase having a viscosity of 15 mPa·s at 25°C

   into contact with the oily stains on the subject surface, and performing rinsing with water or hot water without mixing together the composition and the oily stains on the subject surface.

2. The method according to claim 1, wherein in the composition, a content of the component (A) is from 5 to 50 mass%, and a content of the component (B) is from 50 to 95 mass%.

3. The method according to claim 1 or 2, wherein the oily stains comprise a cosmetic.

4. The method according to claim 3, wherein the cosmetic comprises a foundation.

5. The method according to any one of claims 1 **to 4,** wherein the subject surface is hydrophobic.

6. The method according to any one of claims 1 **to 5,** wherein the subject surface is human skin.

7. The method according to any one of claims 1 **to 6,** wherein the subject surface is rinsed with water or hot water after a lapse of an arbitrary time of from 10 seconds to 20 minutes after the composition is brought into contact with the oily stains on the subject surface.

8. The method according to any one of claims 1 **to 7,** wherein the component (A) in the composition brought into contact with the oily stains on the subject surface infiltrates into the oily stains with the component (B), and makes the subject surface hydrophilic, and then the subject surface is rinsed with water or hot water to remove the oily stains from the subject surface.

9. A composition comprising the following components (A) and (B):

   (A) from 5 to 50 mass% of a nonionic surfactant having an HLB value of from 7 to 14 and/or a log P value of from 2.5 to 10.8, and
(B) from 50 to 95 mass% of an oil phase having a viscosity of 15 mPa·s at 25°C,

wherein the component (A) infiltrates into target oily stains on a solid surface with the component (B), and makes the subject surface hydrophilic.

10. A composition comprising the following components (A) and (B):

(A) from 5 to 50 mass% of a nonionic surfactant having an HLB value of from 7 to 14 and/or a log P value of from 2.5 to 10.8, and
(B) from 50 to 95 mass% of an oil phase having a viscosity of 15 mPa·s at 25°C,

wherein the component (A) infiltrates into target oily stains on a solid surface with the component (B), and modifies the subject surface to improve water wettability of the subject surface.

11. A composition comprising the following components (A) and (B):

(A) from 5 to 50 mass% of a nonionic surfactant having an HLB value of from 7 to 14 and/or a log P value of from 2.5 to 10.8, and
(B) from 50 to 95 mass% of an oil phase having a viscosity of 15 mPa·s at 25°C,

wherein the component (A) infiltrates into target oily stains on a solid surface with the component (B), and modifies the subject surface to draw water between the subject surface and the oily stains.

Fig. 1

Before cleaning    After cleaning    After cleaning      After cleaning
               with massage   without massage    with massage
                                                magnified by 30
                                                times in DSA

Fig. 2

After cleaning and      After cleaning and
wiping                  wiping
without massage       with massage

## INTERNATIONAL SEARCH REPORT

| International application No. |
|---|
| **PCT/JP2023/038904** |

**A.  CLASSIFICATION OF SUBJECT MATTER**

*A61Q 1/14*(2006.01)i; *A61K 8/39*(2006.01)i
FI:  A61K8/39; A61Q1/14

According to International Patent Classification (IPC) or to both national classification and IPC

**B.  FIELDS SEARCHED**

Minimum documentation searched (classification system followed by classification symbols)

A61Q1/14; A61K8/39

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| X | JP 2014-201523 A (NISSHIN KAGAKU KK) 27 October 2014 (2014-10-27) examples 2, 4-6, claims, paragraphs [0015], [0016], [0031]-[0033] | 1-11 |
| X | JP 2018-027907 A (NISSHIN KAGAKU KK) 22 February 2018 (2018-02-22) examples 4, 5, claims, paragraphs [0028], [0036], [0037] | 1-11 |
| X | JP 2022-533314 A (L'OREAL) 22 July 2022 (2022-07-22) examples, tables 2-3, 6-8, claims, paragraph [0030] | 1, 3-8 |
| A |  | 2, 9-11 |
| X | JP 2022-129357 A (KOSE CORP.) 05 September 2022 (2022-09-05) examples 1-8, 12-21, comparative examples 1-8, claims, paragraph [0030] | 9-11 |
| A |  | 1-8 |
| X | JP 2016-088883 A (PIAS ARISE KK) 23 May 2016 (2016-05-23) examples 1-10, 12, 14-16, comparative examples 1, 2, claims | 9-11 |
| A |  | 1-8 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **21 December 2023** | **16 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**EP 4 613 338 A1**

**INTERNATIONAL SEARCH REPORT**

| International application No. |
| --- |
| **PCT/JP2023/038904** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2021-154602 A (MITSUBISHI PAPER MILLS LTD.) 07 October 2021 (2021-10-07) paragraph [0061] | 1-11 |

Form PCT/ISA/210 (second sheet) (January 2015)

**INTERNATIONAL SEARCH REPORT**
Information on patent family members

International application No.

**PCT/JP2023/038904**

| Patent document cited in search report | | | Publication date (day/month/year) | Patent family member(s) | Publication date (day/month/year) |
|---|---|---|---|---|---|
| JP | 2014-201523 | A | 27 October 2014 | (Family: none) | |
| JP | 2018-027907 | A | 22 February 2018 | (Family: none) | |
| JP | 2022-533314 | A | 22 July 2022 | US 2020/0368558 A1 examples, tables 2-3, 6-8, claims, paragraph [0053] WO 2020/242814 A1 EP 3975976 A1 KR 10-2021-0134653 A CN 113747871 A | |
| JP | 2022-129357 | A | 05 September 2022 | (Family: none) | |
| JP | 2016-088883 | A | 23 May 2016 | (Family: none) | |
| JP | 2021-154602 | A | 07 October 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017100980 A
- JP 2014047141 A
- JP 2006022004 A

**Non-patent literature cited in the description**

- *J. Soc. Comet. Chem. Japan*, 1991, vol. 25 (3)
- VoCE. Kodansha Ltd, October 2022
- **KLAMT et al.** *Journal of Computer-Aided Molecular Design*, 2001, vol. 15, 355-365